(19) Europäisches Patentamt
European Patent Office
Office européen des brevets

(11) Veröffentlichungsnummer: **0 278 414 B1**

(12) **EUROPÄISCHE PATENTSCHRIFT**

(45) Veröffentlichungstag der Patentschrift: **11.08.93**

(51) Int. Cl.[5]: **C07C 209/18**, C07C 211/54, C07C 323/34, C07C 309/63

(21) Anmeldenummer: **88101663.8**

(22) Anmeldetag: **05.02.88**

# (54) Verfahren zur Herstellung von Umsetzungsprodukten des 2,2-Bis (4-hydroxyphenyl) hexafluorpropans sowie solche neuen Derivate.

(30) Priorität: **10.02.87 DE 3704005**

(43) Veröffentlichungstag der Anmeldung:
**17.08.88 Patentblatt 88/33**

(45) Bekanntmachung des Hinweises auf die Patenterteilung:
**11.08.93 Patentblatt 93/32**

(84) Benannte Vertragsstaaten:
**CH DE ES FR GB IT LI NL**

(56) Entgegenhaltungen:

**JOURNAL OF POLYMER SCIENCE, Polymer Chemistry Edition, Band 20, Nr. 9, September 1982, Seiten 2381-2393, New York, US; K.S.Y. LAU: "Synthesis of polymer intermediates containing the hexafluoroisopropylidene group via functionalization of 2,2-Diphenylhexafluoropropane"**

(73) Patentinhaber: **HOECHST AKTIENGESELL-SCHAFT**

**W-6230 Frankturt am Main 80(DE)**

(72) Erfinder: **Schneider, Klaus-Albert, Dr.**
**Schillerring 30**
**W-6234 Hattersheim an Main(DE)**
Erfinder: **Siegemund, Günter, Dr.**
**Frankturter Strasse 21**
**W-6238 Hofheim am Taunus(DE)**

EP 0 278 414 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß 2,2-Bis[3-amino-4-(4-phenoxyanilino)phenyl]hexafluorpropan sowie 2,2-Bis(3-amino-4-anilinophenyl)hexafluorpropan aus 2,2-Bis(4-hydroxyphenyl)hexafluorpropan hergestellt werden können (K.S.Y. LAU, A.L. LANDIS, W.J. KELLEGHAN und C.D. BEARD, J. Polym. Sci., Polym. Chem. Ed. 20, 1982, (9), 2381 - 93 und K.S.Y. LAU, W.J. KELLEGHAN, R.H. BOSCHAN und N. BILOW, J. Polym. Sci., Polym. Chem. Ed. 1983, 21 (10), 3009 - 11, 14 und 21). Dabei werden beide phenolische Gruppen des Ausgangsstoffes 2,2-Bis(4-hydroxyphenyl)hexafluorpropan unter Verwendung des schwierig zu handhabenden und teuren Trifluormethansulfonsäureanhydrides in $CF_3$-$SO_2$-O-(Triflat-)Gruppen umgewandelt. Im anschließenden Verfahrensschritt wird das so hergestellte Di-Triflat mit Nitriersäure bei 100 °C in 2,2-Bis(3-nitro-4-triflatophenyl)hexafluorpropan übergeführt. Damit wurde eine gegenüber Nucleophilen stark aktivierte Stelle im Aromaten geschaffen, denn Triflatgruppen stellen bekanntlich sehr leicht abspaltbare Gruppen dar. Im dritten Verfahrensschritt werden dann die beiden Triflatgruppen in einer nucleophilen Substitutionsreaktion durch je zwei 4-Phenoxyanilino- oder Anilinogruppen ersetzt. Im letzten Verfahrensschritt erfolgt mittels einer katalytischen Reduktion der beiden Nitrogruppen die Freisetzung der entsprechenden aromatischen Tetramine.

Die Nachteile des vorgenannten Verfahrens sind, besonders im Hinblick auf eine Herstellung im technischen Maßstab, die schwer zu beherrschende Umsetzung mit Trifluormethansulfonsäureanhydrid sowie die Nitrierung mit Nitriersäure bei 100 °C. Der Preis des Trifluormethansulfonsäureanhydrides, aber besonders die Handhabung dieser gefährlichen Chemikalie sowie auch der mit Sicherheitsrisiken behaftete Umgang mit heißer Nitriersäure macht eine Verfahrensverbesserung wünschenswert.

Es wurde nun überraschenderweise gefunden, daß die bekannten Verbindungen 2,2-Bis(3-amino-4-anilinophenyl)hexafluorpropan und 2,2-Bis[3-amino-4-(4-phenoxyanilino)phenyl]hexafluorpropan und andere aromatische Tetramine nach einem Verfahren hergestellt werden können, welches die oben erwähnten Nachteile ausschließt. Schlüsselverbindungen dieses neuen Verfahrens sind neue Zwischenprodukte der Formel II (s. Patentanspruch 9), worin $R^3$ Wasserstoff oder $NO_2$ und $R^4$ OMes (1), OTos (2) oder OBros (3) darstellen. Dabei bedeutet Mes den Methansulfonyl-, Tos den Toluolsulfonyl- und Bros den Bromphenylsulfonylrest.

Diejenigen leicht herstellbaren Verbindungen der Formel II, in denen $R^3$ $NO_2$ bedeutet, können mit Nucleophilen, die noch mindestens ein freies Wasserstoffatom haben, umgesetzt werden. Als Nucleophile eignen sich vor allem primäre und sekundäre Amine wie Anilin, Phenoxyanilin und Anilinderivate, die am Kern noch bis zu drei Substituenten mit insgesamt 1 bis 4 Kohlenstoffatomen enthalten, wie Alkylgruppen und/oder eine Alkoxygruppe, oder die andere elektronenschiebende Substituenten wie Brom und/oder Chlor, oder an Stelle dessen auch elektronenziehende Substituenten wie die Trifluormethylgruppe enthalten, sowie Mercaptane. Von den Aminen sind die primären bevorzugt, so daß sich Verbindungen der Formel I (siehe Patentanspruch 1) bilden, in denen $R^2$ ein Aminrest ist, an dessen Stickstoff noch ein Wasserstoffatom gebunden ist. Im einzelnen seien beispielsweise genannt die verschiedenen Toluidine, Xylidine, Äthyl-, Propyl- und Butylaniline oder das Aminoderivat des Cymols, ferner die verschiedenen Methoxy-, Äthoxy-, Propoxy- und Butoxyaniline, und zwar jeweils einschließlich der isomeren Formen der Propyl- und Butylreste, also z.B. das Aminoderivat des Cumols, sowie deren Brom- und Chlorderivate, die diese Halogene am Kern enthalten. Bevorzugt sind die Verbindungen, die unsubstituiert sind oder die nur einen Substituenten enthalten, und von den substituierten insbesondere solche, die den Substituenten in p-Stellung enthalten, wie das 4-Methoxyanilin. Geeignete Amine sind auch das Cyclohexylamin, das Hydrierungsprodukt des Benzylamins und Alkylderivate dieser Amine mit bis zu 10 Kohlenstoffatomen, wobei die Alkylreste also einen oder mehrere der Reste Methyl, Äthyl, n- und iso-Propyl bzw. die verschiedenen Butylreste darstellen. Weitere geeignete Verbindungen sind die Trifluormethylaniline, insbesondere das 4-Trifluormethylanilin, das Benzylamin, Thiophenol und Benzylmercaptan. Es findet in allen Fällen ein nucleophiler Angriff am aromatischen Ring statt - wobei die Mesylat- bzw. Tosylat- bzw. Brosylatgruppen eliminiert werden.

Gegenstand der Erfindung ist also ein Verfahren zur Herstellung von Verbindungen der Formel I

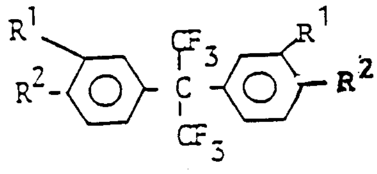

worin bedeuten $R^1$ $NO_2$ oder $NH_2$ und $R^2$ einen Rest eines cycloaliphatischen Amins mit 6 Ringatomen und insgesamt höchstens 10 Kohlenstoffatomen, der also einen oder mehrere der Reste Methyl, Äthyl, n- und iso-Propyl bzw. der verschiedenen Butylreste enthält, einen primären oder sekundären Anilinrest, der unsubstituiert ist oder am Kern bis zu drei Substituenten aus der Gruppe Alkyl, Alkoxy, Chlor und Brom, wovon höchstens zwei Halogen und höchstens einer Alkoxy sein können, und insgesamt höchstens 10 Kohlenstoffatome enthält, oder der eine Trifluormethyl-, Phenoxy- oder Benzylgruppe enthält, den Benzylamino-, Phenylmercapto- oder den Benzylmercaptorest, das dadurch gekennzeichnet ist, daß man

$a_1$) 2,2-Bis(4-hydroxyphenyl)hexafluorpropan mit Mesyl-, Tosyl- bzw, Brosylhalogenid zu den Zwischenprodukten 2,2-Bis(4-mesyloxyphenyl)hexafluorpropan, 2,2-Bis(4-tosyloxyphenyl)hexafluorpropan bzw. 2,2-Bis(4-brosyloxyphenyl)hexafluorpropan (d.s. Verbindungen der Formel II mit $R^3$ = H) umsetzt und diese dann mit Salpetersäure zu den Zwischenstufen 2,2-Bis(4-mesyloxy-3-nitrophenyl)hexafluorpropan, 2,2-Bis(3-nitro-4-tosyloxyphenyl)hexafluorpropan bzw. 2,2-Bis(4-brosyloxy-3-nitrophenyl)hexafluorpropan (d.s. Verbindungen der Formel II mit $R^3$ = $NO_2$) nitriert oder

$a_2$) 2,2-Bis(4-hydroxy-3-nitrophenyl)hexafluorpropan mit Mesyl-, Tosyl- bzw. Brosylhalogenid zu den in $a_1$) genannten Zwischenstufen 2,2-Bis(4-mesyloxy-3-nitrophenyl)hexafluorpropan, 2,2-Bis(3-nitro-4-tosyloxyphenyl)hexafluorpropan bzw. 2,2-Bis(4-brosyloxy-3-nitrophenyl)hexafluorpropan (d.s. Verbindungen der Formel II mit $R^3$ = $NO_2$) umsetzt und

b) diese dann mit dem der Bedeutung von $R^2$ entsprechenden Amin, mit Thiophenol oder Benzylmercaptan zu Verbindungen der Formel I umsetzt, in denen $R^1$ $NO_2$ bedeutet, wobei das Halogen des Mesyl-, Tosyl- und Brosylhalogenids ein Atomgewicht zwischen 35 und 80 hat, also Chlor oder Brom ist, und

c) daß man die nach b) erhaltenen Verbindungen isoliert oder katalytisch zu Verbindungen der Formel I hydriert, in denen $R^2$ $NH_2$ bedeutet.

Gegenstand der Erfindung sind auch Verbindungen der Formel I, worin $R^1$ $NO_2$ oder $NH_2$ ist und $R^2$ einen Rest eines cycloaliphatischen Amins mit 6 Ringatomen und insgesamt höchstens 10 Kohlenstoffatomen, den Benzylamino, Phenylmercapto- oder den Benzylmercaptorest bedeutet. Gegenstand der Erfindung sind ferner Verbindungen der Formel II, worin $R^3$ Wasserstoff oder $NO_2$ und $R^4$ OMes, OTos oder OBros bedeuten, sowie ein Verfahren zu deren Herstellung, das dadurch gekennzeichnet ist, daß man

$\alpha$) 2,2-Bis(4-hydroxyphenyl)hexafluorpropan bzw. 2,2-Bis(4-hydroxy-3-nitrophenyl)hexafluorpropan mit Mesyl-, Tosyl- bzw. Brosylchlorid oder -bromid umsetzt oder

$\beta$ 2,2-Bis(4-mesyloxyphenyl)- oder 2,2-Bis(4-tosyloxyphenyl)- oder 2,2-Bis(4-brosyloxyphenyl)-hexafluorpropan mit Salpetersäure nitriert.

Nach dem erfindungsgemäßen Verfahren erhält man aus dem leicht zugänglichen 2,2-Bis(4-hydroxyphenyl)hexafluorpropan (Bisphenol AF) auf verschiedenen Wegen die neuen 2,2-Bis(4-mesyloxy- bzw. 2,2-Bis(4-tosyloxy- bzw. 2,2-Bis(4-brosyloxy-3-nitrophenyl)hexafluorpropane bzw. die in 3-Stellung jeweils unsubstituierten Analogen als neue, reaktive, für aromatische Substitutionsreaktionen geeignete Zwischenprodukte. Diese stellen besonders vorteilhafte Ausgangsstoffe für die Synthese aromatischer Di- und Tetramine dar.

Es ist ein besonderer Vorteil der Erfindung, daß sich die Nitroverbindungen der Formel II (9, 10 und 11) in nur zwei Verfahrensschritten, ausgehend vom Bisphenol AF, herstellen lassen, und zwar durch Nitrierung mit Salpetersäure und Umsetzung mit Mesyl-, Tosyl- bzw. Brosylhalogenid, vorzugsweise -chlorid, in beliebiger Reihenfolge. Beide Wege sind im Prinzip gleichwertig (s. Schema 1). Bei der einen Möglichkeit wird 2,2-Bis(4-hydroxyphenyl)hexafluorpropan unter Bedingungen, wie sie für die Einführung einer einzigen Nitrogruppe in einen phenolischen Kern üblich sind - bevorzugt mit halbkonzentrierter Salpetersäure - zu 2,2-Bis(4-hydroxy-3-nitrophenyl)hexafluorpropan 8 nitriert und in einer zweiten Verfahrensstufe in üblicher Weise mit dem entsprechenden Säurehalogenid in Gegenwart einer Base, z. B. einem tertiären Amin wie Triäthyl-, Tripropyl-, Triisopropyl und einem der verschiedenen Tributylamine, vorzugsweise Pyridin, zu den Zwischenprodukten 9, 10 und 11 umgesetzt. Alternativ dazu kann man auch 2,2-Bis(4-hydroxyphenyl)-

hexafluorpropan 4 unter üblichen Bedingungen mit dem entsprechenden Säurehalogenid in Gegenwart einer Base und dann unter Bedingungen, wie sie für die Einführung einer einzigen Nitrogruppe in den aromatischen Kern von Estern von Phenolen üblich sind - bevorzugt mit halbkonzentrierter Salpetersäure - zu den gleichen Produkten 9, 10 und 11 umsetzen. Die Ausbeuten der einzelnen Reaktionsschritte liegen alle zwischen 80 und 95 Prozent.

Die 3-Nitroverbindungen der Formel II lassen sich erfindungsgemäß durch nucleophile aromatische Substitutionsreaktionen mit einer Vielzahl von primären und sekundären Aminen, mit Thiophenol oder Benzylmercaptan, die wohlfeil und einfach zu handhaben sind, unter Austausch der Mesyloxy-, Tosyloxy- bzw. Brosyloxygruppen zu einer Reihe von interessanten, überwiegend neuen, im Schema 2 gezeigten aromatischen Verbindungen umsetzen. Stellvertretend werden darin einige Umsetzungen mit 9 aufgeführt. Setzt man die 3-Nitroverbindungen der Formel II mit Anilin bzw. 4-Phenoxyanilin um, so gelangt man nach einer katalytischen Hydrierung zu dem schon bekannten, auf anderem Wege hergestellten 2,2-Bis(3-amino-4-anilinophenyl)hexafluorpropan bzw. 2,2-Bis[3-amino-4-(4-phenoxyanilino)phenyl]hexafluorpropan. Außer zu diesen beiden bekannten Tetraminen führt das erfindungsgemäße Verfahren gemäß Anspruch 1 zu einer Reihe von neuen aromatischen Tetraminen sowie Diaminodimercaptoverbindungen.

Das Verfahren wird besonders vorteilhaft mit Mesylchlorid durchgeführt, da dieses flüssig ist, und ist in den Schemata 1, 2, und 3 beschrieben.

Um zur entsprechenden Tetramino- bzw. Diaminodimercaptoverbindung zu gelangen, werden die in Schema 2 genannten Dinitroverbindungen mit Wasserstoff katalytisch unter üblichen Hydrierungsbedingungen reduziert (vgl. Schema 3), z.B. mit Hilfe von Platin-, Palladium- oder Nickelkatalysatoren auf üblichen Trägern wie Kohlenstoff. Die so hergestellten Zwischenstufen und Endprodukte wie 2,2-Bis(4-mesyloxyphenyl)hexafluorpropan, 2,2-Bis(4-mesyloxy-3-nitrophenyl)hexafluorpropan, die entsprechenden Tosyl- bzw. Brosylanaloga der Formel II

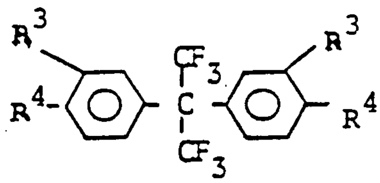

bzw. Verbindungen der allgemeinen Formel I (s. Patentanspruch 2) sind neu. Derartige, fluorierte aromatische Di- bzw. Tetramine stellen wertvolle Monomere für Polybenzimidthiazole und Polybenzimidazole dar.

Die Einführung der Hexafluorisopropylideneinheiten führt bei solchen Polymeren zu einer enormen Verbesserung verschiedener physikalischer bzw. physikalisch-chemischer Eigenschaften, wie der thermischen Stabilität und der dielektrischen Eigenschaften.

Im folgenden wird zunächst die Herstellung des Ausgangsproduktes für die Variante $a_2$) und danach die Durchführung des erfindungsgemäßen Verfahrens und die Herstellung von erfindungsgemäßen Verbindungen beschrieben. Das verwendete Äthanol ist 96 %ig. Smp. bedeutet Schmelzpunkt.

Ausgangsprodukt für die Variante $a_2$)

2,2-Bis(4-hydroxy-3-nitrophenyl)hexafluorpropan

Zu einer Mischung von 302,4 g (0,9 mol) Bisphenol AF und 1000 ml Chloroform werden bei einer Reaktionstemperatur von 15 - 25 °C unter kräftigem Rühren 924 g einer aus 439 ml 98 %iger Salpetersäure und 561 ml Wasser hergestellten verdünnten Salpetersäure getropft. Bei dieser Temperatur wird zur Vervollständigung der Reaktion eine Stunde nachgerührt. Die organische Phase wird anschließend abgetrennt, die wäßrige Phase dreimal mit Dichlormethan extrahiert. Die vereinigten organischen Phasen werden mit Natriumhydrogencarbonat-Lösung neutral gewaschen und über $MgSO_4$ getrocknet. Nach Abziehen des Lösungsmittels werden 427 g eines zähen Öles als Rohprodukt erhalten, aus welchem nach der Umkristallisation aus Äthanol 322 g (84% Ausbeute) 2,2-Bis(4-hydroxy-3-nitrophenyl)hexafluorpropan isoliert werden. Smp. 116 - 119 °C.

IR(KBr): $\nu$ = 1540, 1340 $cm^{-1}$ ($NO_2$), 1280 - 1140 $cm^{-1}$ ($CF_3$).

$^1H(CDCl_3)$: $\delta$ = 10,7 (s, breit; 2 H, Hydroxyl), 8,3 (d, $^4J_{H,H}$ = 2 Hz, 2 H, aromat.), 7,6 (d,d, $^3J_{H,H}$ = 9 Hz, $^4J_{H,H}$ = 2 Hz, 2 H, aromat.), 7,3 (d, $^3J_{H,H}$ = 9 Hz, 2 H, aromat.)

$^{13}C(CDCl_3)$: $\delta$ = 155,5, 138,1, 133,3, 126,9, 124,3, 120,8, (aromat.), 123,4 (q, $^1J_{C,F}$ = 287 Hz, $CF_3$), 63,3

(sept. $^2J_{C,F}$ = 28 Hz, $C(CF_3)_2$).
$^{19}F(CDCl_3)$: δ = -64,7 ($CF_3$).

| | | | | | | |
|---|---|---|---|---|---|---|
| $C_{15}H_8F_6N_2O_6$: (426,2) | Ber.% Gef.% | C 42,3 C 42,4 | H 1,9 H 1,7 | F 26,7 F 26,8 | N 6,6 N 6,4 | O 22,5 O 22,0. |

Beispiele

2,2-Bis(4-mesyloxy-3-nitrophenyl)hexafluorpropan

A) Zu einer Mischung von 21,3 g (0,05 mol) 2,2-Bis(4-hydroxy-3-nitrophenyl)hexafluorpropan und 100 ml Pyridin werden bei einer Reaktionstemperatur von 0 - 5 °C 11,5 g (0,1 mol) Methansulfonsäurechlorid getropft. Es wird 1 Stunde bei 5 °C und über Nacht bei Raumtemperatur nachgerührt. Anschließend wird das Reaktionsgemisch zuerst mit verdünnter, dann mit halbkonzentrierter Salzsäure angesäuert; der ausgefallene Niederschlag wird abgesaugt und mit Wasser neutral gewaschen. Das Rohprodukt (29,3, g) wird dann aus Toluol umkristallisiert, so daß 24,8 g analysenreines 2,2-Bis(4-mesyloxy-3-nitrophenyl)-hexafluorpropan isoliert werden können. Ausbeute 85 %; Smp. 119 - 121 °C.
IR(KBr): ν = 1540, 1340 $cm^{-1}$ ($NO_2$), 1360, 1090 $cm^{-1}$ ($-SO_2O-$), 1280 - 1140 $cm^{-1}$ ($CF_3$).
$^1H(CDCl_3)$: δ = 8,1 (mc, 2 H, aromat.), 7,6 (mc, 4 H, aromat.), 3,4 (s, 6 H, $CH_3$).

| | | | | | | |
|---|---|---|---|---|---|---|
| $C_{17}H_{12}F_6N_2O_{10}S_2$: (582,4) | Ber.% Gef.% | C 35,1 C 35,3 | H 2,1 H 2,1 | F 19,6 F 19,4 | N 4,8 N 5,1 | S 11,0 S 11,3. |

B) Nach einer alternativen Arbeitsweise werden 68,9 g (0,14 mol) 2,2-Bis(4-mesyloxyphenyl)-hexafluorpropan bei einer Reaktionstemperatur von 0 - 10 °C portionsweise in 90 ml 98 %ige Salpetersäure eingetragen. Anschließend wird über Nacht bei Raumtemperatur nachgerührt. Nach der Hydrolyse mit Eiswasser wird die organische Phase abgetrennt und die wäßrige Phase viermal mit Dichlormethan extrahiert. Die vereinigten organischen Phasen werden mit verdünnter Natriumhydrogencarbonatlösung neutral gewaschen und über $MgSO_4$ getrocknet. Nach Abziehen des Lösungsmittels und Umkristallisation werden 75 g (92 % Ausbeute) 2,2-Bis(4-mesyloxy-3-nitrophenyl)hexafluorpropan erhalten.

2) 2,2-Bis(4-mesyloxyphenyl)hexafluorpropan

Zu einer Lösung von 16,8 g (0,05 mol) Bisphenol AF in 100 ml Pyridin werden bei einer Reaktionstemperatur von 0 - 5 °C 11,8 g (0,1 mol) Methansulfonsäurechlorid getropft. Anschließend wird noch eine Stunde bei 5 °C und über Nacht bei Raumtemperatur nachgerührt. Dann wird die Reaktionsmischung mit so viel halbkonzentrierter Salzsäure angesäuert, bis das Produkt ausfällt. Nach Absaugen und Waschen mit Wasser werden 24 g Rohprodukt, nach der Umkristillisation aus Toluol 20,2 g (82 % Ausbeute) 2,2-Bis(4-mesyloxyphenyl)hexafluorpropan isoliert. Smp. 136 - 138 °C.
IR(KBr): ν = 1370 $cm^{-1}$ ($-SO_2O-$), 1290 - 1150 $cm^{-1}$ ($CF_3$).
$^1H(CDCl_3)$: δ = 7,4 (mc, 8 H, aromat.), 3,2 (s, 6 H, $CH_3$).

| | | | | | |
|---|---|---|---|---|---|
| $C_{17}H_{14}F_6O_6S_2$: (492,4) | Ber.% Gef.% | C 41,5 C 41,4 | H 2,9 H 2,7 | F 23,1 F 23,0 | S 13,0 S 13,4 |

3) 2,2-Bis(3-nitro-4-tosyloxyphenyl)hexafluorpropan

Eine Lösung von 42,6 g (0,1 mol) 2,2-Bis(4-hydroxy-3-nitrophenyl)hexafluorpropan und 39,1 g (0,205 mol) p-Toluolsulfonsäurechlorid in 300 ml Methylenchlorid wird mit 11,5 g (0,205 mol) Kaliumhydroxid versetzt. Die Reaktionsmischung wird dann fünf Stunden bei Raumtemperatur gerührt, wobei sie sich orangegelb verfärbt. Nach dem Abtrennen von ausgefallenem Kaliumchlorid (16 g) wird das Filtrat über $MgSO_4$ getrocknet. Nach Abziehen des Lösungsmittels und Umkristallisation aus Toluol werden 43 g 2,2-Bis(3-nitro-4-tosyloxyphenyl)hexafluorpropan isoliert. Ausbeute 58 %; Smp. 206 - 208 °C.

$^1H(DMSO)$: $\delta$ = 8 - 7,3 (m, 14 H, aromat.), 2,4 (s, 6 H, 2 $CH_3$)
$^{19}F(DMSO)$: $\delta$ = -63,1 (s, 2 $CF_3$).

| | | | | | | |
|---|---|---|---|---|---|---|
| $C_{29}H_{20}F_6N_2O_{10}S_2$: (734,6) | Ber.% Gef.% | C 47,4 C 48,0 | H 2,7 H 2,9 | N 3,8 N 3,8 | S 8,7 S 8,2 | F 15,5 F 15,8. |

4) 2,2-Bis(4-anilino-3-nitrophenyl)hexafluorpropan - Herstellung

A) Eine Lösung von 60 g (0,103 mol) 2,2-Bis(4-mesyloxy-3-nitrophenyl)hexafluorpropan und 122,8 g (1,32 mol) Anilin in 400 ml Acetonitril wird 24 Stunden unter Rückfluß erhitzt. Dabei fällt das Methansulfonsäuresalz des Anilins aus, das durch Absaugen abgetrennt wird. Das Filtrat wird durch eine Destillation unter vermindertem Druck vom Lösungsmittel und vom überschüssigen Anilin befreit. Nach der Umkristallisation des Rückstandes aus Äthanol werden 47,3 g 2,2-Bis(4-anilino-3-nitrophenyl)-hexafluorpropan (80 % Ausbeute) erhalten. Smp. 165-166 °C
IR(KBr): $\nu$ = 3370 $cm^{-1}$ (breit, NH), 1520, 1340 $cm^{-1}$ ($NO_2$), 1280 - 1150 $cm^{-1}$ ($CF_3$).
$^1H(CDCl_3)$: $\delta$ = 9,6 (s, breit, 2 H, NH), 8,3 (mc, 2 H, aromat.), 7,3 (mc, 14 H, aromat.).

| | | | | | | |
|---|---|---|---|---|---|---|
| $C_{27}H_{18}F_6N_4O_4$ (576,5) | Ber.% Gef.% | C 56,2 C 56,0 | H 3,1 H 3,1 | F 19,8 F 19,7 | N 9,7 N 9,4 | O 11,1 O 11,4. |

B) Nach einer alternativen Arbeitsweise werden 7,3 g (0,01 mol) 2,2-Bis(3-nitro-4-tosyloxyphenyl)-hexafluorpropan, 11,6 g (0,125 mol) Anilin und 50 ml Acetonitril 24 Stunden unter Rückfluß erhitzt. Nach Erkalten der rotorangenen Suspension wird der Feststoff abgetrennt und mit Acetonitril gewaschen. Das Filtrat wird nach Abziehen des Lösungsmittels zweimal aus Äthanol umkristallisiert. Erhalten werden dann 6,3 g 2,2-Bis(4-anilino-3-nitrophenyl)hexafluorpropan (65 % Ausbeute).

5) 2,2-Bis(3-amino-4-anilinophenyl)hexafluorpropan - Herstellung

In einem 500 ml Autoklaven aus Chromnickelstahl werden 34,6 g (0,06 mol) 2,2-Bis(4-anilino-3-nitrophenyl)hexafluorpropan in 200 ml Äthanol unter der katalytischen Wirkung von 2 g Pd/C (5 Gew.-% - Aktivkohle) mit Wasserstoff reduziert. Nach Filtration und zweimaliger Umkristallisation des Rohproduktes (30,7 g - H - NMR rein) werden 15,1 g analysenreines 2,2-Bis(3-amino-4- anilinophenyl)hexafluorpropan erhalten (49 % Ausbeute). Der Schmelzpunkt beträgt 177 - 178 °C und weicht von dem in der Literatur angegebenen Schmelzpunkt (120 °C) ab.
IR(KBr): $\nu$ = 3550 - 2350 $cm^{-1}$ (breit, NH, $NH_2$), 1280 - 1120 $cm^{-1}$ ($CF_3$).
$^1H(CDCl_3)$: $\delta$ = 7,0 (mc, 16 H, aromat.), 5,3 (s, breit, 2 H, NH), 3,7 (s, breit, 4 H, $NH_2$).

| | | | | | |
|---|---|---|---|---|---|
| $C_{27}H_{22}F_6N_4$: (516,5) | Ber.% Gef.% | C 62,8 C 63,0 | H 4,3 H 4,3 | F 22,1 F 22,4 | N 10,8 N 10,8. |

6) 2,2-Bis(4-benzylamino-3-nitrophenyl)hexafluorpropan

Zu einer Lösung von 58,4 g (0,1 mol) 2,2-Bis(4-mesyloxy-3-nitrophenyl)hexafluorpropan in 700 ml Acetonitril wird bei einer Reaktionstemperatur von 20 - 30 °C eine Lösung von 107,1 g (1 mol) Benzylamin in 100 ml Acetonitril getropft. Nach Abklingen der schwach exothermen Reaktion wird zur Vervollständigung der Reaktion noch 4 Stunden unter Rückfluß gekocht. Beim Erkalten der Reaktionsmischung fällt das Produkt aus. Nach Absaugen und Umkristallisation aus Äthanol werden 40,7 g (67 % Ausbeute) 2,2-Bis(4-benzylamino-3-nitrophenyl)hexafluorpropan erhalten. Smp. 168 - 172 °C. Dieser wurde in einem verschlossenen Schmelzrohr ermittelt, um Sublimation zu vermeiden.
IR(KBr): $\nu$ = 3610 - 2250 $cm^{-1}$ (NH), 1535, 1350 $cm^{-1}$ ($NO_2$), 1290 - 1110 $cm^{-1}$ ($CF_3$).
$^1H(DMSO)$: $\delta$ = 7,8 (d, $^4J_{H,H}$ = 2 Hz, 2 H, aromat.), 7,4 (mc, 10 H, aromat.), 7,0 (dd, $^3J_{H,H}$ = 10 Hz, $^4J_{H,H}$ = 2 Hz, 2 H, aromat.), 6,6 (d, $^3J_{H,H}$ = 10 Hz, 2 H, aromat.), 4,0 (s, 4 H, $CH_2$).
$^{19}F(DMSO)$: $\delta$ = -63,4 ($CF_3$).

| | | |
|---|---|---|
| $C_{29}H_{22}F_6N_4O_4$: | hochaufgelöster Molionenpeak: | Ber. 604,1535<br>Gef. 604,1545. |

7) 2,2-Bis(4-cyclohexylamino-3-nitrophenyl)hexafluorpropan

Die Herstellung erfolgte analog Beispiel 6. Smp. 205 - 208 °C, Ausbeute 90 %.
IR(KBr): $\nu$ = 3450 $cm^{-1}$ (breit, NH), 1530, 1360 $cm^{-1}$ ($NO_2$), 1300 - 1120 $cm^{-1}$ ($CF_3$).
$^1H$(DMSO): $\delta$ = 7,8 (d, $^4J_{H,H}$ = 2 Hz, 2 H, aromat.), 7,4 (s, breit, 2 H, NH), 6,9 (dd, $^3J_{H,H}$ = 10 Hz, $^4J_{H,H}$ = 2 Hz, 2 H, aromat.), 6,5 (d, $^3J_{H,H}$ = 10 Hz, 2 H, aromat.), 1,5 (mc, 22 H, aliphat.).
$^{19}F$(DMSO): $\delta$ = -63,4 ($CF_3$).

8) 2,2-Bis(4-phenylmercapto-3-nitrophenyl)hexafluorpropan

Zu einer Lösung von 29,1 g (0,05 mol) 2,2-Bis(4-mesyloxy-3-nitrophenyl)hexafluorpropan in 10,1 g (0,1 mol) Triäthylamin und 60 ml Dichlormethan wird bei einer Reaktionstemperatur von 20 bis 30 ° C eine Lösung von 11,0 g (0,1 mol) Thiophenol in 40 ml Dichlormethan getropft. Es wird über Nacht nachgerührt. Die Reaktionsmischung wird dann hydrolysiert und dreimal mit Dichlormethan extrahiert. Die vereinigten organischen Phasen werden nach Waschen mit Wasser und Trocknen über $MgSO_4$ durch eine Destillation vom Lösungsmittel befreit. Der Rückstand wird aus Äthanol umkristallisiert (74 % Ausbeute). Smp. 176 - 178 °C
IR(KBr): $\nu$ = 1520, 1340 $cm^{-1}$ ($NO_2$), 1300 - 1110 $cm^{-1}$ ($CF_3$).
$^1H$($CDCl_3$): $\delta$ = 8,2 (d, $^4J_{H,H}$ = 2 Hz, 2 H, aromat.), 7,6 (mc, 10 H, aromat.), 7,3 (d, breit, $^3J_{H,H}$ = 8 Hz, 2 H, aromat.), 6,8 (d, $^3J_{H,H}$ = 8 Hz, 2 H, aromat.).
$^{19}F$($CDCl_3$): $\delta$ = -64,6 ($CF_3$).

9) 2,2-Bis(4-benzylmercapto-3-nitrophenyl)hexafluorpropan

Zu einer Suspension von 13,6 g (0,11 mol) Benzylmercaptan und 15,2 g (0,11 mol) $K_2CO_3$ in 100 ml Acetonitril wird bei Raumtemperatur eine Lösung von 29,7 g (0,051 mol) 2,2-Bis(4-mesyloxy-3-nitrophenyl)-hexafluorpropan getropft. Die Reaktion ist schwach exotherm. Da die Reaktionsmischung im Verlauf der Reaktion immer zäher wird, muß diese mit weiteren 50 ml Acetonitril verdünnt werden. Zur Vervollständigung der Reaktion wird über Nacht nachgerührt. Anschließend wird die Reaktionsmischung hydrolysiert und dann zuerst mit Dichlormethan und danach mit Diäthyläther extrahiert. Die vereinigten organischen Phasen werden über $MgSO_4$ getrocknet. Nach Abziehen des Lösungsmittels und Umkristallisation des Rohproduktes aus Äthanol werden 21,5 g (66 % Ausbeute) 2,2-Bis(4-benzylmercapto-3-nitrophenyl)hexafluorpropan erhalten. Smp. 129 - 130 °C.
IR(KBr): $\nu$ = 1520, 1325 $cm^{-1}$ ($NO_2$), 1280 - 1150 $cm^{-1}$ ($CF_3$).
$^1H$($CDCl_3$): $\delta$ = 8,3 (s, breit, 2 H, aromat.), 7,5 (mc, 14 H, aromat.), 4,3 (s, 4 H, $CH_3$).

| | | | | | | |
|---|---|---|---|---|---|---|
| $C_{29}H_{20}F_6N_2O_4S_2$:<br>(638,6) | Ber.%<br>Gef.% | C 54,5<br>C 54,5 | H 3,1<br>H 2,9 | F 17,8<br>F 17,5 | N 4,4<br>N 4,4 | S 10,0<br>S 10,4 |

10) 2,2-Bis(3-amino-4-benzylmercaptophenyl)hexafluorpropan

Die Herstellung erfolgte analog der im Beispiel 5 beschriebenen katalytischen Reduktion mit Pd/C.
IR(KBr): $\nu$ = 3600 - 2600 $cm^{-1}$ ($NH_2$), 1300 - 1120 $cm^{-1}$ ($CF_3$).
$^1H$($CDCl_3$): $\delta$ = 7,2 (mc, 12 H, aromat.), 6,6 (mc, 4 H, aromat.), 4,2 (s, breit, 4 H, $NH_2$), 3,9 (s, 4 H, $CH_2$).
$^{19}F$($CDCl_3$): $\delta$ = -63,8 ($CF_3$).

Schema 1

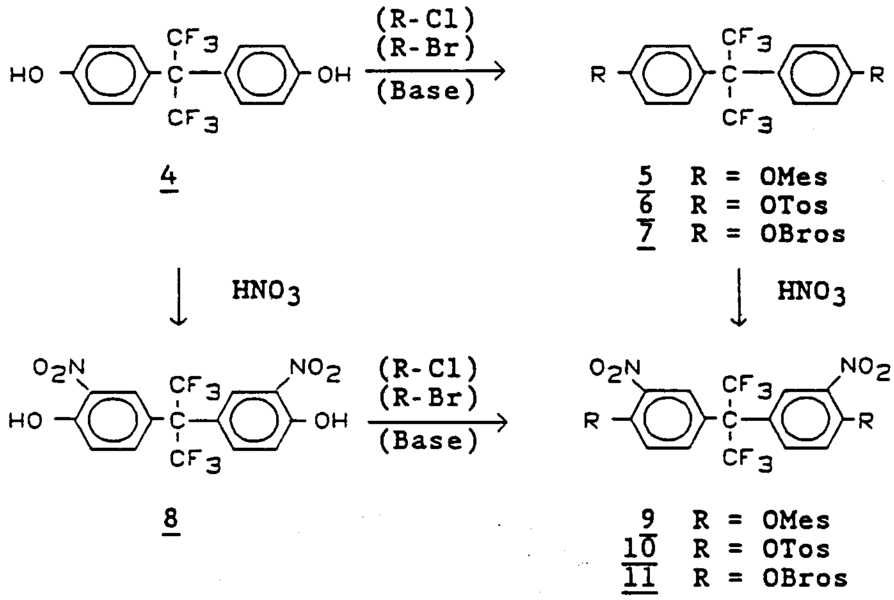


Schema 2

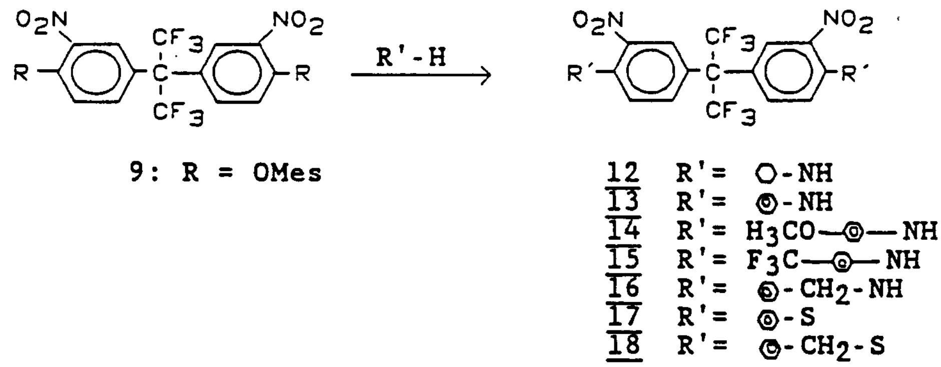


Schema 3

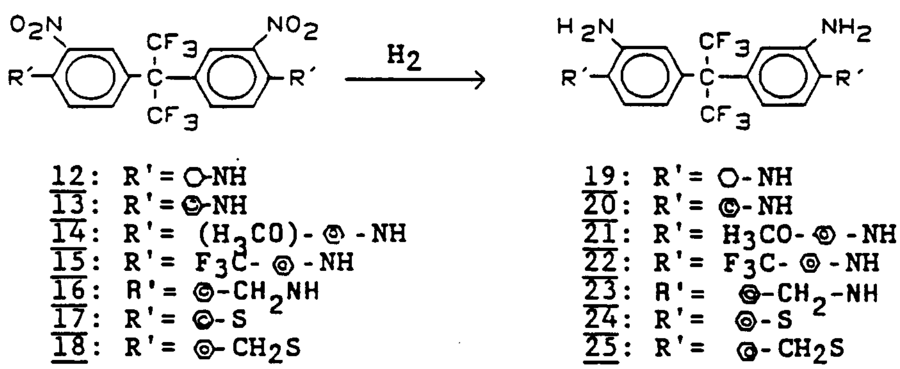

## Patentansprüche

## Patentansprüche für folgende Vertragsstaaten : CH, DE, FR, GB, IT, LI, NL

1. Verfahren zur Herstellung von Verbindungen der Formel I,

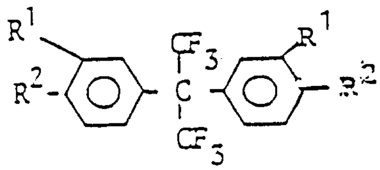

worin bedeuten

$R^1$ $NO_2$ oder $NH_2$ und

$R^2$ einen Rest eines cycloaliphatischen Amins mit 6 Ringatomen und insgesamt höchstens 10 Kohlenstoffatomen, einen primären oder sekundären Anilinrest, der unsubstituiert ist oder am Kern bis zu drei Substituenten aus der Gruppe Alkyl, Alkoxy, Chlor und Brom, wovon höchstens zwei Halogen und höchstens einer Alkoxy sein können, und insgesamt höchstens 10 Kohlenstoffatome enthält, oder der eine Trifluormethyl-, Phenoxy- oder Benzylgruppe enthält, den Benzylamino-, Phenylmercapto- oder den Benzylmercaptorest,

dadurch gekennzeichnet, daß man

$a_1$) 2,2-Bis(4-hydroxyphenyl)hexafluorpropan mit Mesyl-, Tosyl- bzw. Brosylhalogenid zu den Zwischenprodukten 2,2-Bis(4-mesyloxyphenyl)hexafluorpropan, 2,2-Bis(4-tosyloxyphenyl)-hexafluorpropan bzw. 2,2-Bis(4-brosyloxyphenyl)hexafluorpropan umsetzt und diese dann mit Salpetersäure zu den Zwischenstufen 2,2-Bis(4-mesyloxy-3-nitrophenyl)hexafluorpropan, 2,2-Bis(3-nitro-4-tosyloxyphenyl)hexafluorpropan bzw. 2,2-Bis(4-brosyloxy-3-nitrophenyl)hexafluoropropan nitriert, oder

$a_2$) 2,2-Bis(4-hydroxy-3-nitrophenyl)hexafluorpropan mit Mesyl-, Tosyl- bzw. Brosylhalogenid zu den in $a_1$) genannten Zwischenstufen 2,2-Bis(4-mesyloxy-3-nitrophenyl)hexafluorpropan, 2,2-Bis(3-nitro-4-tosyloxyphenyl)hexafluorpropan bzw. 2,2-Bis(4-brosyloxy-3-nitrophenyl)hexafluorpropan umsetzt und

b) diese dann mit dem der Bedeutung von $R^2$ entsprechenden Amin, mit Thiophenol oder Benzylmercaptan zu Verbindungen der Formel I umsetzt, in denen $R^1$ $NO_2$ bedeutet,

wobei das Halogen des Mesyl-, Tosyl- und Brosylhalogenids ein Atomgewicht zwischen 35 und 80 hat, und

c) daß man die nach b) erhaltenen Verbindungen isoliert oder katalytisch zu Verbindungen der Formel I hydriert, in denen $R^2$ $NH_2$ bedeutet.

2. Verbindungen der Formel I, wie im Anspruch 1 angegeben, worin $R^1$ $NO_2$ oder $NH_2$ ist und $R^2$ einen Rest eines cycloaliphatischen Amins mit 6 Ringatomen und insgesamt höchstens 10 Kohlenstoffatomen, den Benzylamino-, Phenylmercapto- oder den Benzylmercaptorest bedeutet.

3. Ausführungsform nach Anspruch 1 oder 2, dadurch gekennzeichnet, daß $R^2$ ein Aminrest ist, wobei an den Stickstoff noch ein Wasserstoffatom gebunden ist.

4. Ausführungsform nach Anspruch 1, 2 oder 3, dadurch gekennzeichnet, daß die Cyclohexylgruppe und die Anilinogruppe höchstens einen Substituenten am Ring enthalten, wobei ein Substituent bevorzugt in p-Stellung steht.

5. Ausführungsform nach einem oder mehreren der Ansprüche 1 bis 4, dadurch gekennzeichnet, daß $R^2$ der Cyclohexylamino, Benzylamino-, 4-Methoxyanilino-, 4-Trifluormethylanilino- oder Benzylanilinorest ist.

6. Ausführungsform nach Anspruch 1 oder 2, dadurch gekennzeichnet, daß $R^2$ der Phenylmercapto- oder Benzylmercaptorest ist.

7. Ausführungsform nach einem oder mehreren der Ansprüche 1 und 3 bis 6, dadurch gekennzeichnet, daß Mesyl-, Tosyl- oder Brosylchlorid verwendet wird.

**8.** Ausführungsform nach Anspruch 7, dadurch gekennzeichnet, daß Mesylchlorid verwendet wird.

**9.** Verbindungen der Formel II

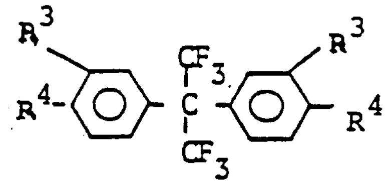

worin $R^3$ Wasserstoff oder $NO_2$ und $R^4$, OTos oder OBros, vorzugsweise aber OMes bedeuten.

**10.** Verfahren zur Herstellung von Verbindungen der Formel II, wie im Anspruch 9 definiert, dadurch gekennzeichnet, daß man

α) 2,2-Bis(4-hydroxyphenyl)hexafluorpropan bzw. 2,2-Bis(4-hydroxy-3-nitrophenyl)hexafluorpropan mit Mesyl-, Tosyl- bzw. Brosylchlorid oder -bromid umsetzt oder

β) 2,2-Bis(4-mesyloxyphenyl)- oder 2,2-Bis(4-tosyloxyphenyl)- oder 2,2-Bis(4-brosyloxyphenyl)-hexafluorpropan mit Salpetersäure nitriert.

**Patentansprüche für folgenden Vertragsstaat : ES**

**1.** Verfahren zur Herstellung von Verbindungen der Formel I,

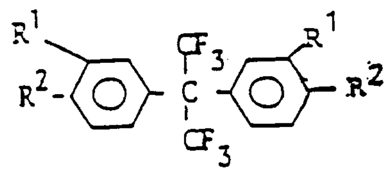

worin bedeuten

$R^1$ $NO_2$ oder $NH_2$ und

$R^2$ einen Rest eines cycloaliphatischen Amins mit 6 Ringatomen und insgesamt höchstens 10 Kohlenstoffatomen, einen primären oder sekundären Anilinrest, der unsubstituiert ist oder am Kern bis zu drei Substituenten aus der Gruppe Alkyl, Alkoxy, Chlor und Brom, wovon höchstens zwei Halogen und höchstens einer Alkoxy sein können, und insgesamt höchstens 10 Kohlenstoffatome enthält, oder der eine Trifluormethyl-, Phenoxy- oder Benzylgruppe enthält, den Benzylamino-, Phenylmercapto- oder den Benzylmercaptorest,

dadurch gekennzeichnet, daß man

$a_1$) 2,2-Bis(4-hydroxyphenyl)hexafluorpropan mit Mesyl-, Tosyl- bzw. Brosylhalogenid zu den Zwischenprodukten 2,2-Bis(4-mesyloxyphenyl)hexafluorpropan, 2,2-Bis(4-tosyloxyphenyl)-hexafluorpropan bzw. 2,2-Bis(4-brosyloxyphenyl)hexafluorpropan umsetzt und diese dann mit Salpetersäure zu den Zwischenstufen 2,2-Bis(4-mesyloxy-3-nitrophenyl)hexafluorpropan, 2,2-Bis(3-nitro-4-tosyloxyphenyl)hexafluorpropan bzw. 2,2-Bis(4-brosyloxy-3-nitrophenyl)hexafluoropropan nitriert, oder

$a_2$) 2,2-Bis(4-hydroxy-3-nitrophenyl)hexafluorpropan mit Mesyl-, Tosyl- bzw. Brosylhalogenid zu den in $a_1$) genannten Zwischenstufen 2,2-Bis(4-mesyloxy-3-nitrophenyl)hexafluorpropan, 2,2-Bis(3-nitro-4-tosyloxyphenyl)hexafluorpropan bzw. 2,2-Bis(4-brosyloxy-3-nitrophenyl)hexafluorpropan umsetzt und

b) diese dann mit dem der Bedeutung von $R^2$ entsprechenden Amin, mit Thiophenol oder Benzylmercaptan zu Verbindungen der Formel I umsetzt, in denen $R^1$ $NO_2$ bedeutet,

wobei das Halogen des Mesyl-, Tosyl- und Brosylhalogenids ein Atomgewicht zwischen 35 und 80 hat, und

c) daß man die nach b) erhaltenen Verbindungen isoliert oder katalytisch zu Verbindungen der Formel I hydriert, in denen $R^2$ $NH_2$ bedeutet.

**2.** Verfahren nach Anspruch 1 zur Herstellung von Verbindungen der Formel I, wie im Anspruch 1 angegeben, dadurch gekennzeichnet, daß $R^1$ $NO_2$ oder $NH_2$ ist und $R^2$ einen Rest eines cycloaliphatischen Amins mit 6 Ringatomen und insgesamt höchstens 10 Kohlenstoffatomen, den Benzylamino-, Phenylmercapto- oder den Benzylmercaptorest bedeutet.

**3.** Verfahren nach Anspruch 1 oder 2, dadurch gekennzeichnet, daß $R^2$ ein Aminrest ist, wobei an den Stickstoff noch ein Wasserstoffatom gebunden ist.

**4.** Verfahren nach Anspruch 1, 2 oder 3, dadurch gekennzeichnet, daß die Cyclohexylgruppe und die Anilinogruppe höchstens einen Substituenten am Ring enthalten, wobei ein Substituent bevorzugt in p-Stellung steht.

**5.** Verfahren nach einem oder mehreren der Ansprüche 1 bis 4, dadurch gekennzeichnet, daß $R^2$ der Cyclohexylamino, Benzylamino-, 4-Methoxyanilino-, 4-Trifluormethylanilino- oder Benzylanilinorest ist.

**6.** Verfahren nach Anspruch 1 oder 2, dadurch gekennzeichnet, daß $R^2$ der Phenylmercapto- oder Benzylmercaptorest ist.

**7.** Verfahren nach einem oder mehreren der Ansprüche 1 und 3 bis 6, dadurch gekennzeichnet, daß Mesyl-, Tosyl- oder Brosylchlorid verwendet wird.

**8.** Verfahren nach Anspruch 7, dadurch gekennzeichnet, daß Mesylchlorid verwendet wird.

**9.** Verfahren zur Herstellung von Verbindungen der Formel II,

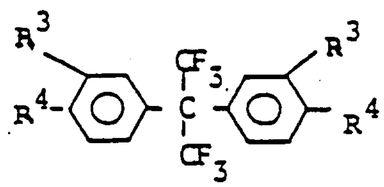

dadurch gekennzeichnet, daß man

α) 2,2-Bis(4-hydroxyphenyl)hexafluorpropan bzw. 2,2-Bis(4-hydroxy-3-nitrophenyl)hexafluorpropan mit Mesyl-, Tosyl- bzw. Brosylchlorid oder -bromid umsetzt oder

β) 2,2-Bis(4-mesyloxyphenyl)- oder 2,2-Bis(4-tosyloxyphenyl)- oder 2,2-Bis(4-brosyloxyphenyl)-hexafluorpropan mit Salpetersäure nitriert.

## Claims

**Claims for the following Contracting States : CH, DE, FR, GB, IT, LI, NL**

**1.** A process for the preparation of compounds of formula I

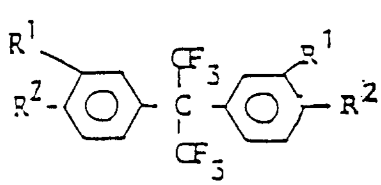

in which

$R^1$ denotes $NO_2$ or $NH_2$ and

$R^2$ denotes a radical of a cycloaliphatic amine having 6 ring atoms and a total of not more than 10 carbon atoms, or a primary or secondary aniline radical which is unsubstituted or contains on the ring up to 3 substituents from the group comprising alkyl, alkoxy, chlorine and bromine, not more than two of which can be halogen and not more than one of which can be

alkoxy, and contains a total of not more than 10 carbon atoms, or which contains a trifluoromethyl, phenoxy or benzyl group, or $R^2$ denotes the benzylamino, phenylmercapto or benzylmercapto radical, which comprises

$a_1$) reacting 2,2-bis-(4-hydroxyphenyl)-hexafluoropropane with a mesyl, tosyl or brosyl halide to give the intermediates 2,2-bis-(4-mesyloxyphenyl)-hexafluoropropane, 2,2-bis-4-tosyloxyphenyl)-hexafluoropropane or 2,2-bis-(4-brosyloxyphenyl)-hexafluoropropane, respectively, and then nitrating these compounds with nitric acid to give the intermediates 2,2-bis-(4-mesyloxy-3-nitrophenyl)-hexafluoropropane, 2,2-bis-(3-nitro-4-tosyloxyphenyl)-hexafluoropropane or 2,2-bis-(4-brosyloxy-3-nitrophenyl)-hexafluoropropane, or

$a_2$) reacting 2,2-bis-(4-hydroxy-3-nitrophenyl)-hexafluoropropane with a mesyl, tosyl or brosyl halide to give the intermediates, mentioned in $a_1$), 2,2-bis-(4-mesyloxy-3-nitrophenyl)-hexafluoropropane,2,2-bis-(3-nitro-4-tosyloxyphenyl)-hexafluoropropane or 2,2-bis-(4-brosyloxy-3-nitrophenyl)-hexafluoropropane, respectively, and

b) then reacting these compounds with the amine corresponding to the meaning of $R^2$, with thiophenol or benzyl mercaptan to give the compounds of the formula I in which $R^1$ denotes $NO_2$, the halogen of the mesyl, tosyl and brosyl halide having an atomic weight between 35 and 80, and

c) isolating the compounds obtained in accordance with b) or hydrogenating them catalytically to give compounds of the formula I in which $R^2$ denotes $NH_2$.

**2.** A compound of the formula I, as indicated in claim 1, in which $R^1$ is $NO_2$ or $NH_2$ and $R^2$ denotes a radical of a cycloaliphatic amine having 6 ring atoms and a total of not more than 10 carbon atoms, or the benzylamino, phenylmercapto or the benzylmercapto radical.

**3.** The embodiment as claimed in claim 1 or 2, wherein $R_2$ is an amine radical in which a hydrogen atom is still attached to the nitrogen.

**4.** The embodiment as claimed in claim 1, 2 or 3, wherein the cyclohexyl group and the anilino group contain at most one substituent on the ring, a substituent preferably being in the p-position.

**5.** The embodiment as claimed in one or more of claims 1 to 4, wherein $R^2$ is the cyclohexylamino, benzylamino, 4-methoxyanilino, 4-trifluoromethylanilino or benzylanilino radical.

**6.** The embodiment as claimed in claim 1 or 2, wherein $R^2$ is the phenylmercapto or benzylmercapto radical.

**7.** The embodiment as claimed in one or more of claims 1 and 3 to 6, wherein mesyl chloride, tosyl chloride or brosyl chloride is used.

**8.** The embodiment as claimed in claim 7, wherein mesyl chloride is used.

**9.** A compound of the formula II

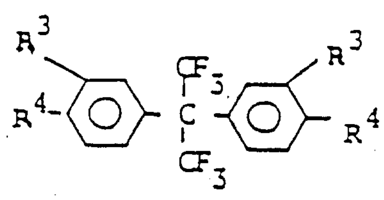

in which $R^3$ is hydrogen or $NO_2$, and $R^4$ is OTos or OBros, but preferably OMes.

**10.** A process for the preparation of a compound of formula II as defined in claim 9, which comprises

α) reacting 2,2-bis-(4-hydroxyphenyl)-hexafluoropropane or 2,2-bis-(4-hydroxy-3-nitrophenyl)-hexafluoropropane with mesyl, tosyl or brosyl chloride or bromide or

β) nitrating 2,2-bis-(4-mesyloxyphenyl)- or 2,2-bis-(4-tosyloxyphenyl)- or 2,2-bis-(4-brosyloxyphenyl)-hexafluoropropane with nitric acid.

**Claims for the following Contracting State : ES**

1. A process for the preparation of compounds of formula I

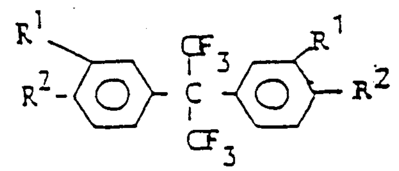

in which

$R^1$ denotes $NO_2$ or $NH_2$ and

$R^2$ denotes a radical of a cycloaliphatic amine having 6 ring atoms and a total of not more than 10 carbon atoms, or a primary or secondary aniline radical which is unsubstituted or contains on the ring up to 3 substituents from the group comprising alkyl, alkoxy, chlorine and bromine, not more than two of which can be halogen and not more than one of which can be alkoxy, and contains a total of not more than 10 carbon atoms, or which contains a trifluoromethyl, phenoxy or benzyl group, or $R^2$ denotes the benzylamino, phenylmercapto or benzylmercapto radical, which comprises

$a_1$) reacting 2,2-bis-(4-hydroxyphenyl)-hexafluoropropane with a mesyl, tosyl or brosyl halide to give the intermediates 2,2-bis-(4-mesyloxyphenyl)-hexafluoropropane, 2,2-bis-4-tosyloxyphenyl)-hexafluoropropane or 2,2-bis-(4-brosyloxyphenyl)-hexafluoropropane, respectively, and then nitrating these compounds with nitric acid to give the intermediates 2,2-bis-(4-mesyloxy-3-nitrophenyl)-hexafluoropropane, 2,2-bis-(3-nitro-4-tosyloxyphenyl)-hexafluoropropane or 2,2-bis-(4-brosyloxy-3-nitrophenyl)-hexafluoropropane, or

$a_2$) reacting 2,2-bis-(4-hydroxy-3-nitrophenyl)-hexafluoropropane with a mesyl, tosyl or brosyl halide to give the intermediates, mentioned in $a_1$), 2,2-bis-(4-mesyloxy-3-nitrophenyl)-hexafluoropropane,2,2-bis-(3-nitro-4-tosyloxyphenyl)-hexafluoropropane or 2,2-bis-(4-brosyloxy-3-nitrophenyl)-hexafluoropropane, respectively, and

b) then reacting these compounds with the amine corresponding to the meaning of $R^2$, with thiophenol or benzyl mercaptan to give the compounds of the formula I, in which $R^1$ denotes $NO_2$, the halogen of the mesyl, tosyl and brosyl halide having an atomic weight between 35 and 80, and

c) isolating the compounds obtained in accordance with b) or hydrogenating them catalytically to give compounds of the formula I in which $R^2$ denotes $NH_2$.

2. A process as claimed in claim 1 for preparing a compound of the formula I, as indicated in claim 1, wherein $R^1$ is $NO_2$ or $NH_2$ and $R^2$ denotes a radical of a cycloaliphatic amine having 6 ring atoms and a total of not more than 10 carbon atoms, or the benzylamino, phenylmercapto or the benzylmercapto radical.

3. The process as claimed in claim 1 or 2, wherein $R^2$ is an amine radical in which a hydrogen atom is still attached to the nitrogen.

4. The process as claimed in claim 1, 2 or 3, wherein the cyclohexyl group and the anilino group contain at most one substituent on the ring, a substituent preferably being in the p-position.

5. The process as claimed in one or more of claims 1 to 4, wherein $R^2$ is the cyclohexylamino, benzylamino, 4-methoxyanilino, 4-trifluoromethylanilino or benzylanilino radical.

6. The process as claimed in claim 1 or 2, wherein $R^2$ is the phenylmercapto or benzylmercapto radical.

7. The process as claimed in one or more of claims 1 and 3 to 6, wherein mesyl chloride, tosyl chloride or brosyl chloride is used.

8. The process as claimed in claim 7, wherein mesyl chloride is used.

**9.** A process for the preparation of a compound of the formula II

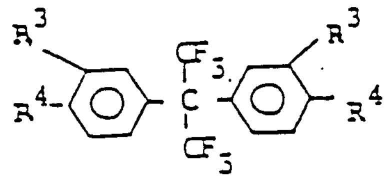

wherein

α) 2,2-bis-(4-hydroxyphenyl)-hexafluoropropane or 2,2-bis-(4-hydroxy-3-nitrophenyl)-hexafluoropropane is reacted with mesyl, tosyl or brosyl chloride or bromide or

β) 2,2-bis-(4-mesyloxyphenyl)- or 2,2-bis-(4-tosyloxyphenyl)- or 2,2-bis-(4-brosyloxyphenyl)-hexafluoropropane is nitrated with nitric acid.

**Revendications**
**Revendications pour les Etats contractants suivants : CH, DE, FR, GB, IT, LI, NL**

**1.** Procédé pour préparer des composés de formule I,

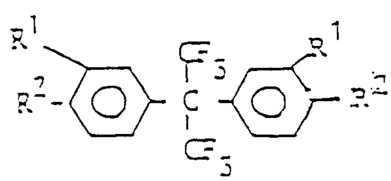

dans laquelle

$R^1$ est $NO_2$ ou $NH_2$ et

$R^2$ est le résidu d'une amine cycloaliphatique ayant 6 atomes dans le cycle, et en tout 10 atomes de carbone, un résidu aniline primaire ou secondaire, lequel est non substitué ou contient sur le noyau jusqu'à trois substituants choisis parmi l'ensemble comprenant les radicaux alkyle, alcoxy, chloro et bromo, dont au plus deux substituants peuvent être des halogènes et dont au plus un substituant peut être un radical alcoxy, et en tout au plus 10 atomes de carbone, ou encore qui contient un groupe trifluorométhyle, phénoxy ou benzyle, ou encore l'un des radicaux benzylamino, phénylmercapto ou benzylmercapto,

caractérisé en ce que

$a_1$) on fait réagir du bis(hydroxy-4 phényl)-2,2 hexafluoropropane avec de l'halogénure de mésyle, de tosyle ou de brosyle pour donner les produits intermédiaires respectivement bis(mésyloxy-4 phényl)-2,2 hexafluoropropane, bis(tosyloxy-4 phényl)-2,2 hexafluoropropane et bis(brosyloxy-4 phényl)-2,2 hexafluoropropane, puis qu'on nitre ces derniers à l'aide d'acide nitrique pour obtenir les stades intermédiaires respectivement bis(mésyloxy-4 nitro-3 phényl)-2,2 hexafluoropropane, bis-(nitro-3 tosyloxy-4 phényl)-2,2 hexafluoropropane et bis(brosyloxy-4 nitro-3 phényl)-2,2 hexafluoropropane, ou bien

$a_2$) on fait réagir le bis(hydroxy-4 nitro-3 phényl)-2,2 hexafluoropropane avec un halogénure de mésyle, de tolyle et de brosyle, pour obtenir les stades intermédiaires mentionnés en $a_1$) ci-dessus, respectivement bis(mésyloxy-4 nitro-3 phényl)-2,2 hexafluoropropane, bis(nitro-3 tosyloxy-4 phényl)-2,2 hexafluoropropane et bis(brosyloxy-4 nitro-3 phényl)-2,2 hexafluoropropane, puis

b) on fait réagir ces derniers avec l'amine correspondant à la signification de $R^2$, avec du thiophénol ou du benzylmercaptan, pour obtenir des composés de formule I dans lesquels $R^1$ est $NO_2$,

l'halogène de l'halogénure de mésyle, de tosyle et de brosyle ayant une masse atomique de 35 à 80, et

c) en ce qu'on isole les composés obtenus en b), ou encore qu'on les hydrogène par voie catalytique pour obtenir des composés de formule I où $R^2$ est $NH_2$.

**2.** Composés de formule I selon la revendication 1, dans lesquels $R^1$ est $NO_2$ ou $NH_2$, et $R^2$ est le résidu d'une amine cycloaliphatique ayant 6 atomes sur le cycle et en tout 10 atomes de carbone, le radical

benzylamino, phénylmercapto ou benzylmercapto.

**3.** Forme de réalisation selon les revendications 1 ou 2, caractérisée en ce que $R^2$ est un résidu amine, un atome d'hydrogène étant encore fixé à l'azote.

**4.** Forme de réalisation selon les revendications 1, 2 ou 3, caractérisée en ce que le groupe cyclohexyle et le groupe anilino contiennent au plus un substituant sur le noyau, un substituant étant de préférence en position para.

**5.** Forme de réalisation selon l'une ou plusieurs des revendications 1 à 4, caractérisée en ce que $R^2$ est le radical cyclohexylamino, benzylamino, méthoxy-4 anilino, trifluorométhyl-4 anilino ou benzylanilino.

**6.** Forme de réalisation selon les revendications 1 ou 2, caractérisée en ce que $R^2$ est le radical phénylmercapto ou benzylmercapto.

**7.** Forme de réalisation selon l'une ou plusieurs des revendications 1 et 3 à 6, caractérisée en ce qu'on utilise du chlorure de mésyle, de tosyle ou de brosyle.

**8.** Forme de réalisation selon la revendication 7, caractérisée en ce qu'on utilise du chlorure de mésyle.

**9.** Composés de formule II

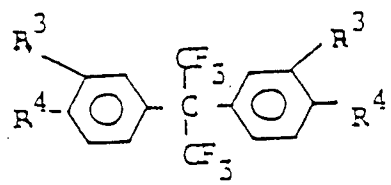

dans laquelle $R^3$ est un hydrogène ou $NO_2$, et $R^4$ est OTos ou OBros, mais de préférence OMes.

**10.** Procédé pour préparer des composés de formule II selon la revendication 9, caractérisé en ce que
α) on fait réagir du bis(hydroxy-4 phényl)-2,2 hexafluoropropane ou du bis(hydroxy-4 nitro-3 phényl)-2,2 hexafluoropropane avec du chlorure ou du bromure de mésyle, de tosyle ou de brosyle, ou encore
β) on nitre du bis(mésyloxy-4 phényl)-2,2- ou du bis(tosyloxy-4 phényl)-2,2- ou du bis(brosyloxy-4 phényl)-2,2 hexafluoropropane à l'aide d'acide nitrique.

**Revendications pour l'Etat contractant suivant : ES**

**1.** Procédé pour préparer des composés de formule I,

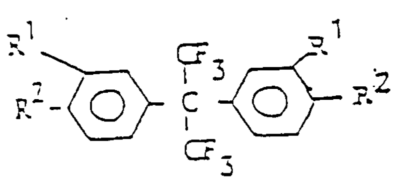

dans laquelle
$R^1$ est $NO_2$ ou $NH_2$ et
$R^2$ est le résidu d'une amine cycloaliphatique ayant 6 atomes dans le cycle, et en tout 10 atomes de carbone, un résidu aniline primaire ou secondaire, lequel est non substitué ou contient sur le noyau jusqu'à trois substituants choisis parmi l'ensemble comprenant les radicaux alkyle, alcoxy, chloro et bromo, dont au plus deux substituants peuvent être des halogènes et dont au plus un substituant peut être un radical alcoxy, et en tout au plus 10 atomes de carbone, ou encore qui contient un groupe

trifluorométhyle, phénoxy ou benzyle, ou encore l'un des radicaux benzylamino, phénylmercapto ou benzylmercapto,

caractérisé en ce que

$a_1$) on fait réagir du bis(hydroxy-4 phényl)-2,2 hexafluoropropane avec de l'halogénure de mésyle, de tosyle ou de brosyle pour donner les produits intermédiaires respectivement bis(mésyloxy-4 phényl)-2,2 hexafluoropropane, bis(tosyloxy-4 phényl)-2,2 hexafluoropropane et bis(brosyloxy-4 phényl)-2,2 hexafluoropropane, puis qu'on nitre ces derniers à l'aide d'acide nitrique pour obtenir les stades intermédiaires respectivement bis(mésyloxy-4 nitro-3 phényl)-2,2 hexafluoropropane, bis-(nitro-3 tosyloxy-4 phényl)-2,2 hexafluoropropane et bis(brosyloxy-4 nitro-3 phényl)-2,2 hexafluoropropane, ou bien

$a_2$) on fait réagir le bis(hydroxy-4 nitro-3 phényl)-2,2 hexafluoropropane avec un halogénure de mésyle, de tolyle et de brosyle, pour obtenir les stades intermédiaires mentionnés en $a_1$) ci-dessus, respectivement bis(mésyloxy-4 nitro-3 phényl)-2,2 hexafluoropropane, bis(nitro-3 tosyloxy-4 phényl)-2,2 hexafluoropropane et bis(brosyloxy-4 nitro-3 phényl)-2,2 hexafluoropropane, puis

b) on fait réagir ces derniers avec l'amine correspondant à la signification de $R^2$, avec du thiophénol ou du benzylmercaptan, pour obtenir des composés de formule I dans lesquels $R^1$ est $NO_2$,

l'halogène de l'halogénure de mésyle, de tosyle et de brosyle ayant une masse atomique de 35 à 80, et

c) en ce qu'on isole les composés obtenus en b), ou encore qu'on les hydrogène par voie catalytique pour obtenir des composés de formule I où $R^2$ est $NH_2$.

**2.** Procédé selon la revendication 1, pour préparer des composés de formule I selon la revendication 1, caractérisé en ce que $R^1$ est $NO_2$ ou $NH_2$, et $R^2$ est le résidu d'une amine cycloaliphatique ayant 6 atomes sur le cycle et en tout 10 atomes de carbone, le radical benzylamino, phénylmercapto ou benzylmercapto.

**3.** Procédé selon les revendications 1 ou 2, caractérisé en ce que $R^2$ est un résidu amine, un atome d'hydrogène étant encore fixé à l'azote.

**4.** Procédé selon les revendications 1, 2 ou 3, caractérisé en ce que le groupe cyclohexyle et le groupe anilino contiennent au plus un substituant sur le noyau, un substituant étant de préférence en position para.

**5.** Procédé selon l'une ou plusieurs des revendications 1 à 4, caractérisé en ce que $R^2$ est le radical cyclohexylamino, benzylamino, méthoxy-4 anilino, trifluorométhyl-4 anilino ou benzylanilino.

**6.** Procédé selon les revendications 1 ou 2, caractérisé en ce que $R^2$ est le radical phénylmercapto ou benzylmercapto.

**7.** Procédé selon l'une ou plusieurs des revendications 1 et 3 à 6, caractérisé en ce qu'on utilise du chlorure de mésyle, de tosyle ou de brosyle.

**8.** Procédé selon la revendication 7, caractérisé en ce qu'on utilise du chlorure de mésyle.

**9.** Procédé pour préparer des composés de formule II

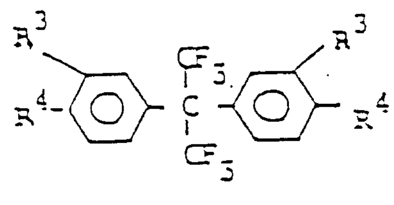

caractérisé en ce que

α) on fait réagir du bis(hydroxy-4 phényl)-2,2 hexafluoropropane ou du bis(hydroxy-4 nitro-3 phényl)-2,2 hexafluoropropane avec du chlorure ou du bromure de mésyle, de tosyle ou de brosyle, ou encore

β) on nitre du bis(mésyloxy-4 phényl)-2,2 ou du bis(tosyloxy-4 phényl)-2,2 ou du bis(brosyloxy-4 phényl)-2,2 hexafluoropropane à l'aide d'acide nitrique.